# FASCICULE DE BREVET EUROPEEN

(11) **EP 2 680 961 B1**
(45) Date de publication et mention de la délivrance du brevet: **31.01.2018**
(21) Numéro de dépôt: 12704318.0
(22) Date de dépôt: 01.02.2012
(51) Int. Cl.: C12M 3/06, C12M 3/00, G01N 35/08, B01L 3/00, B01J 19/00, A61K 6/00, C12M 1/12, C12Q 1/02

(54) **PROCÉDÉ DE SUIVI DE RÉACTION ET SYSTÈME RÉACTIONNEL POUR SA MISE EN UVRE**
VERFAHREN ZUR ÜBERWACHUNG EINER REAKTION UND REAKTIONSSYSTEM ZUR DURCHFÜHRUNG DES VERFAHRENS
METHOD FOR MONITORING A REACTION, AND REACTION SYSTEM FOR IMPLEMENTING SAME

(30) Priorité: 04.03.2011 FR 1100659
(43) Date de publication de la demande: 08.01.2014
(73) Titulaire: Centre National de la Recherche Scientifique, 75016 Paris (FR); Université Pierre et Marie Curie (Paris 6), 75005 Paris (FR)
(72) Inventeur: BERTHOLLE, Fabien, 93370 Montfermeil (FR); BIBETTE, Jérôme, 75006 Paris (FR); BAUDRY, Jean-Marie, 75011 Paris (FR); BREMOND, Nicolas, 75005 Paris (FR); BARABAN, Larysa, 75014 Paris (FR); PANIZZA, Pascal, 35530 Noyal-sur-Vilaine (FR)
(74) Mandataire: Lavoix
(86) Numéro de dépôt international: PCT/IB2012/050478
(87) Numéro de publication internationale: WO 2012/120384

(56) Documents cités:
- WO-A1-2004/089533
- WO-A2-2007/021818
- US-A1- 2009 042 319
- ATENCIA ET AL.: "Controlled microfluidic interfaces", NATURE, vol. 437, 29 septembre 2005 (2005-09-29), pages 648-655, XP002662053, cité dans la demande

## Description

L'invention se rapporte à un procédé de suivi de réaction et à un système réactionnel pour sa mise en oeuvre.

L'étude d'entités microbiologiques, telles que des micro-organismes uni ou pluricellulaires, nécessite de pouvoir détecter une population de ces micro-organismes dans un ou plusieurs réacteurs, ainsi que le développement de ces micro-organismes au cours du temps. Il est également nécessaire de pouvoir sélectionner facilement et d'extraire les populations d'intérêt.

Il existe actuellement des machines appelées "plate reader" (lecteurs de plaques en français) qui se présentent sous la forme de plaques munies d'une multitude de puits (jusqu'à 1536 actuellement sur une surface de 127.76 x 85.48 mm) agencées selon une matrice en deux dimensions, et dans lesquelles les micro-organismes sont mis en culture dans un milieu de culture donné.

Le premier inconvénient de ce type de machine est la nécessité d'un système de balayage à deux dimensions pour faire coïncider le puits que l'on souhaite remplir ou analyser avec l'outil adapté (dispositif de remplissage ou outil de mesure). Un tel dispositif est coûteux, volumineux et doit être très précis pour assurer une bonne coïncidence entre chaque puits et l'outil.

Un autre inconvénient majeur est la nécessité de prendre des précautions importantes lors de la manipulation de la plaque pour éviter de faire vibrer ou de renverser la plaque et de créer des contaminations entre les puits. Or, il faut constamment agiter les puits pour garder un milieu homogène et dans le cas des micro-organismes pour éviter la formation de biofilms. Cela sous-entend que la quantité de biomasse atteinte ou détectable dans un puits est limitée.

Un autre inconvénient est la difficulté à maitriser l'évaporation du milieu de culture dans les puits, compte-tenu du faible volume de liquide contenu dans chaque puits. Une solution proposée est de maintenir la plaque sous atmosphère contrôlée et/ou de réapprovisionner régulièrement chaque puits en milieu de culture pour compenser l'évaporation.

De tels procédés sont fastidieux et peuvent influencer la croissance des micro-organismes, induisant un biais dans les expérimentations et les mesures.

Dans l'article « Controlled microfluidic interfaces » (Atencia & Beebe, Nature Review, 437, 648-655 2005), il a déjà été proposé de réaliser un système réactionnel comprenant :
- plusieurs réservoirs de solutions de réactifs reliés de manière fluidique à un tube capillaire d'injection ;
- au moins un réservoir d'un fluide porteur non miscible avec les solutions de réactifs, relié de manière fluidique à un tube capillaire de réaction, le tube capillaire d'injection étant monté débouchant dans le tube capillaire de réaction de sorte que des gouttes individuelles de réactifs puissent être injectées dans le tube capillaire de réaction, au sein du fluide porteur non miscible, de manière à former une pluralité de réacteurs.

Ce système permet de réaliser de nombreuses expériences à la suite, notamment pour étudier les facteurs intervenant dans la cristallisation des protéines. Une fois la mesure réalisée (mesure de diffraction par rayons X), les gouttes sont véhiculées vers l'extrémité du tube de réaction et éliminées.

Une autre application consiste à réaliser l'encapsulation d'agents thérapeutiques. Une fois cette encapsulation faite, on récupère les gouttes à la sortie du tube de réaction et on les conditionne pour utilisation.

Ce système résout de nombreux problèmes posés par les lecteurs de plaque.

Cependant, ce système ne permet pas de faire un suivi de la cinétique des réactions, en particulier de l'évolution temporelle de culture de cellules vivantes ainsi que de les trier. La présente invention vise à résoudre les inconvénients précédents et propose un système réactionnel, en particulier de cultures de micro-organismes, économique, peu encombrant, facile à mettre en oeuvre, permettant de contrôler totalement le milieu de culture tout au long de l'expérimentation, et permettant un suivi de la cinétique des réactions, en particulier de l'évolution temporelle de culture de cellules vivantes et leur tri.

Pour cela, l'invention propose de réaliser un système réactionnel capillaire, notamment pour la culture de micro-organismes, comprenant un moyen de référencement des gouttes pour les identifier de manière unique dans le train de gouttes, et au moins un moyen de mise en recirculation des réacteurs devant au moins un capteur de suivi de réaction.

A cette fin, l'invention a pour objet un système réactionnel, notamment de cultures de micro-organismes, comprenant :
- au moins un réservoir de milieu réactionnel relié de manière fluidique à un tube capillaire d'injection ;
- au moins un réservoir d'un fluide porteur non miscible avec le milieu réactionnel, relié de manière fluidique à un tube capillaire de réaction,
- le tube capillaire d'injection étant monté débouchant dans le tube capillaire de réaction de sorte que des gouttes individuelles de milieu réactionnel puissent être injectées dans le tube capillaire de réaction, au sein du fluide porteur non miscible, de manière à former un train de réacteurs,
- au moins un détecteur de suivi de réaction,

Le système réactionnel selon l'invention comprend un moyen de référencement des réacteurs pour les identifier de manière unique dans le train de réacteurs, et au moins un moyen de mise en recirculation des réacteurs devant au moins un détecteur de suivi de réaction.

Selon d'autres modes de réalisation :
- le moyen de mise en recirculation peut comprendre une boucle de recirculation des réacteurs devant le ou les détecteurs, cette boucle de recirculation comprenant un capillaire débouchant en amont et en aval du ou des détecteurs ;
- le moyen de mise en recirculation peut comprendre un moyen de mise en recirculation apte à inverser le sens de circulation des réacteurs ;
- le milieu réactionnel peut être un milieu de culture de microorganismes, le tube capillaire de réaction étant alors un tube capillaire de culture, et les réacteurs étant des réacteurs de culture de micro-organismes ;
- le système réactionnel peut comprendre, en outre, au moins un réservoir d'un fluide dit « de séparation » non miscible avec le fluide porteur et non miscible avec le milieu réactionnel, relié de manière fluidique au tube capillaire de réaction de sorte que des gouttes de fluide de séparation puissent être injectées dans le fluide porteur entre deux réacteurs ;
- le système réactionnel peut comprendre, en outre, au moins un réservoir de réactif relié de manière fluidique au tube capillaire d'injection et/ou au tube capillaire de réaction, de sorte que du réactif puisse être mélangé au milieu réactionnel ;
- le fluide porteur et le fluide de séparation peuvent être des huiles non miscibles entre elles, le milieu réactionnel étant un milieu aqueux non miscible avec les huiles précitées ;
- le système réactionnel peut comprendre, en outre, au moins un réservoir de déchet, en liaison fluidique avec le tube capillaire de réaction ;
- le système réactionnel peut comprendre, en outre, au moins un détecteur, le tube capillaire de réaction comprenant au moins une partie transparente à un signal émis et/ou capté par le détecteur ;
- le système réactionnel peut comprendre, en outre, un tube capillaire de prélèvement monté débouchant dans le tube capillaire de réaction de sorte qu'au moins un réacteur puisse être prélevé ;
- le système réactionnel peut comprendre, en outre, au moins un tube capillaire de dérivation monté débouchant dans le tube capillaire de réaction de sorte qu'au moins un réacteur puissent être dérivé vers un moyen de traitement d'un ou plusieurs réacteurs ;
- le système réactionnel peut comprendre, en outre, une unité centrale de contrôle reliée au moyen de mise en circulation et apte à :
   ∘ commander l'injection de gouttes individuelles de milieu réactionnel dans le tube capillaire de réaction, au sein du fluide porteur, en imposant une vitesse et une durée de circulation du milieu de réactionnel, de manière à former une pluralité de réacteurs ;
   ∘ commander la circulation du fluide porteur en imposant une vitesse, une durée et un sens de circulation du fluide porteur dans le tube capillaire de réaction ;
   ∘ compter les réacteurs dans le milieu porteur et mémoriser la place de chaque réacteur par rapport à un réacteur de référence ;
   ∘ faire recirculer les réservoirs dans le tube capillaire de réaction ;
- l'unité centrale de contrôle peut être apte à commander l'injection de gouttes de fluide de séparation entre deux réacteurs ;
- l'unité centrale de contrôle peut être apte à commander l'injection d'au moins un réactif au sein du milieu réactionnel pour en modifier la composition et/ou les propriétés chimiques et/ou physiques ; et/ou
- l'unité centrale de contrôle peut être, en outre, reliée au détecteur et est apte à mémoriser au moins une mesure réalisée par le ou les détecteur(s).

L'invention se rapporte également à un procédé de suivi de réaction dans un milieu réactionnel, comprenant les étapes suivantes :
a) remplir un tube capillaire de réaction avec un milieu porteur non miscible avec le milieu réactionnel ;
b) injecter, grâce à un tube capillaire d'injection, une goutte individuelle de milieu réactionnel dans le tube capillaire de réaction, au sein du fluide porteur non miscible ;
c) mettre en circulation le fluide porteur pour que la goutte de milieu réactionnel se déplace par rapport au tube capillaire d'injection ;
e) répéter les étapes b) et c) pour créer un train ordonné de gouttes de milieu réactionnel au sein du fluide porteur pour former une pluralité de réacteurs ;
f) mesurer au moins un paramètre représentatif de chaque réacteur ;
g) faire recirculer au moins un réacteur pour mesurer ledit au moins un paramètre représentatif au cours du temps.

Selon d'autres modes de réalisation ;
- le procédé peut comprendre les étapes suivantes :
   a) remplir un tube capillaire de culture avec un milieu porteur non miscible avec le milieu de culture ;
   b) injecter, grâce à un tube capillaire d'injection, une goutte individuelle de milieu de culture dans le tube capillaire de culture, au sein du fluide porteur non miscible ;
   c) mettre en circulation le fluide porteur pour que la goutte de milieu de culture se déplace par rapport au tube capillaire d'injection ;
   e) répéter les étapes b) et c) pour créer un train ordonné de gouttes de milieu de culture au sein du fluide porteur pour former une pluralité de réacteurs de culture de micro-organismes ;
   f) mesurer au moins un paramètre représentatif de chaque réacteur de culture, ce paramètre pouvant être représentatif de la quantité de micro-organismes présents dans chaque réacteur ;
   g) faire recirculer au moins un réacteur pour mesurer ledit au moins un paramètre représentatif au cours du temps.
- le procédé peut comprendre, en outre, après l'étape c) et avant l'étape e), une étape d) comprenant l'injection, dans le fluide porteur, d'une goutte d'un fluide dit « de séparation », non miscible avec le fluide porteur et non miscible avec le milieu réactionnel, de sorte qu'au moins une goutte de fluide de séparation soit intercalée entre deux réacteurs et empêche leur coalescence ;
- la mesure peut être réalisée par un procédé optique tel que des mesures d'absorbance, de diffusion ou de fluorescence, ou par une mesure électrique telle que l'impédance ; et/ou
- le procédé peut comprendre, en outre, après l'étape e), une étape f') de récupération d'au moins un réacteur d'intérêt par aspiration dudit réacteur d'intérêt dans un tube capillaire de prélèvement monté débouchant dans le tube capillaire de réaction.

D'autres caractéristiques de l'invention seront énoncées dans la description détaillée ci-après, faite en référence aux figures annexées qui représentent, respectivement :
- la figure 1, une vue schématique en coupe partielle d'une première partie fonctionnelle du système réactionnel selon l'invention ;
- la figure 2, une photo en agrandissement d'une coupe partielle d'un tube capillaire de réactions selon l'invention ;
- la figure 3, une vue schématique en coupe partielle d'une deuxième partie fonctionnelle du système réactionnel selon l'invention, selon un premier mode de réalisation ;
- la figure 4, une vue schématique en coupe partielle d'une deuxième partie fonctionnelle du système réactionnel selon l'invention, selon un deuxième mode de réalisation ;
- la figure 5, une vue schématique en coupe partielle d'une deuxième partie fonctionnelle du système réactionnel selon l'invention, selon un troisième mode de réalisation ; et
- la figure 6, un exemple de courbes de mesures de suivi de la croissance de populations de micro-organismes dans un système réactionnel selon l'invention.

Dans la description détaillée ci-après, le système réactionnel décrit est un système de culture de micro-organismes ou de cellules biologiques vivantes. Dans cette application, le milieu réactionnel est un milieu de culture de microorganismes. Les autres réactifs peuvent être des nutriments, des solutions pour modifier le pH, etc.

Néanmoins, la structure décrite peut être utilisée dans d'autres domaines (chimique ou expérimental) pour suivre des réactions dans le temps.

Dans la description détaillée ci-après, un tube capillaire est un tube fluidique à l'échelle millimétrique, c'est-à-dire présentant un diamètre intérieur de l'ordre du dixième de millimètre au millimètre, de préférence compris entre 0,5 et 1mm. Par exemple, on peut utiliser pour mettre en oeuvre la présente invention, des connectiques et des tubes capillaires pour chromatographie. Un mode de réalisation préféré utilise des tubes de 0,5 millimètre de diamètre, permettant d'obtenir des gouttes de milieu de culture d'environ 100nL.

L'invention propose un procédé de culture de micro-organismes dans un milieu de culture comprenant les étapes suivantes :
a) remplir un tube capillaire de réaction avec un milieu porteur non miscible avec le milieu réactionnel ;
b) injecter, grâce à un tube capillaire d'injection, une goutte individuelle de milieu réactionnel dans le tube capillaire de réaction, au sein du fluide porteur non miscible ;
c) mettre en circulation le fluide porteur pour que la goutte de milieu réactionnel se déplace par rapport au tube capillaire d'injection ;
e) répéter les étapes b) et c) pour créer un train ordonné de gouttes de milieu réactionnel au sein du fluide porteur pour former une pluralité de réacteurs ;
f) mesurer au moins un paramètre représentatif de chaque réacteur ou un signal issu de l'activité de ces micro-organismes ;
g) faire recirculer au moins un réacteur pour mesurer le ou les paramètre(s) représentatif(s) au cours du temps. Cette recirculation peut être des allers-retours et/ou des passages successifs du train de gouttes devant le détecteur grâce au moyen de mise en recirculation, pour la mesure au cours du temps de la quantité de micro-organismes

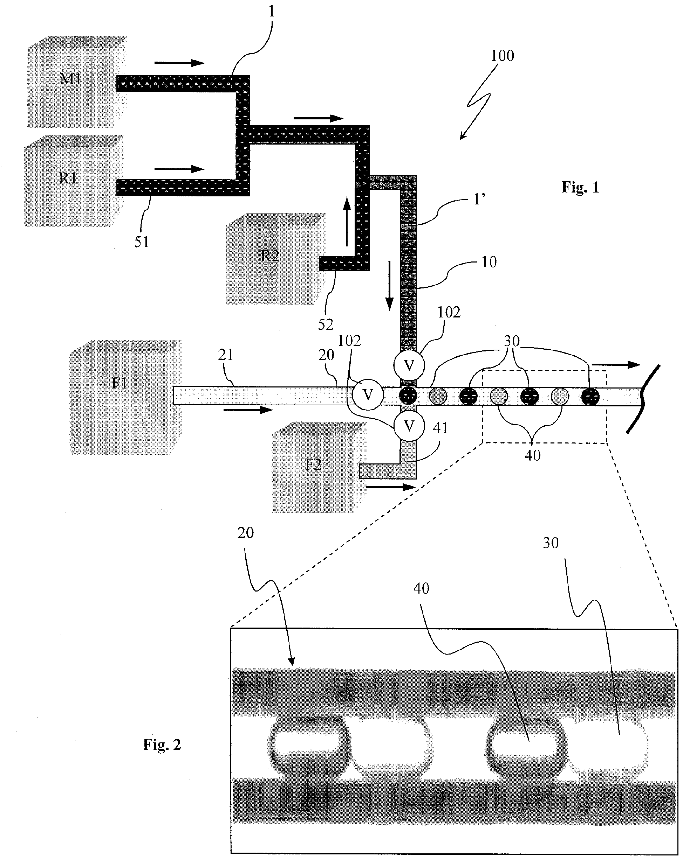

Pour mettre en oeuvre ce procédé, l'invention propose un système réactionnel 100 de cultures de micro-organismes dont une première partie fonctionnelle est illustrée en figure 1.

Le système réactionnel 100 de cultures comprend un ou plusieurs réservoir(s) M1 de milieu de culture 1 relié de manière fluidique à un tube capillaire d'injection 10, Cette liaison fluidique se fait par l'intermédiaire de connexions en T.

Le tube capillaire d'injection 10 est monté débouchant dans un tube capillaire de culture 20 par l'intermédiaire d'une vanne à deux voies 102.

Au moins un réservoir F1 d'un fluide porteur 21 non miscible avec le milieu de culture 1, est relié de manière fluidique au tube capillaire de culture 20 par l'intermédiaire d'une vanne à deux voies 102.

Le système réactionnel 100 de cultures selon l'invention comprend également au moins un moyen de mise en circulation du milieu de culture 1, du fluide porteur 21 et de tout autre fluide utilisé dans le système réactionnel 100 selon l'invention.

Ce moyen de mise en circulation est apte à générer un débit dans les différents capillaires et à commander les vannes à deux voies 102 de l'ensemble du système réactionnel 100.

Avantageusement, le moyen de mise en circulation permet de générer un débit dans les deux sens au sein d'au moins certains capillaires. Autrement dit, il est capable d'inverser le sens de circulation du fluide porteur, et donc des réservoirs, dans certains des capillaires.

L'agencement débouchant du tube capillaire d'injection 10 dans le tube capillaire de culture 20 permet l'injection, par le moyen de mise en circulation, de gouttes individuelles 30 de milieu de culture 1 dans le tube capillaire de culture 20, au sein du fluide porteur 21 non miscible avec le milieu de culture 1. Avantageusement, le tube d'injection est monté débouchant dans le tube de culture par l'intermédiaire de jonctions, tel qu'une jonction en T ou une jonction en croix, équipée d'une ou plusieurs vanne(s) adaptée(s).

Le fluide porteur 21 est avantageusement une huile alors que le milieu de culture 1 est aqueux.

Il est ainsi possible de concevoir des gouttes d'émulsion inverse (eau dans huile) mono dispersées en contrôlant les débits (ou la pression) des fluides immiscibles. En imposant une vitesse et une durée de circulation du milieu de culture et/ou du fluide porteur, il est possible d'injecter précisément un volume déterminé de milieu de culture dans le fluide porteur sous forme de gouttes individuelles.

Chaque goutte 30 constitue un réacteur de culture de micro-organismes au sein du fluide porteur 21.

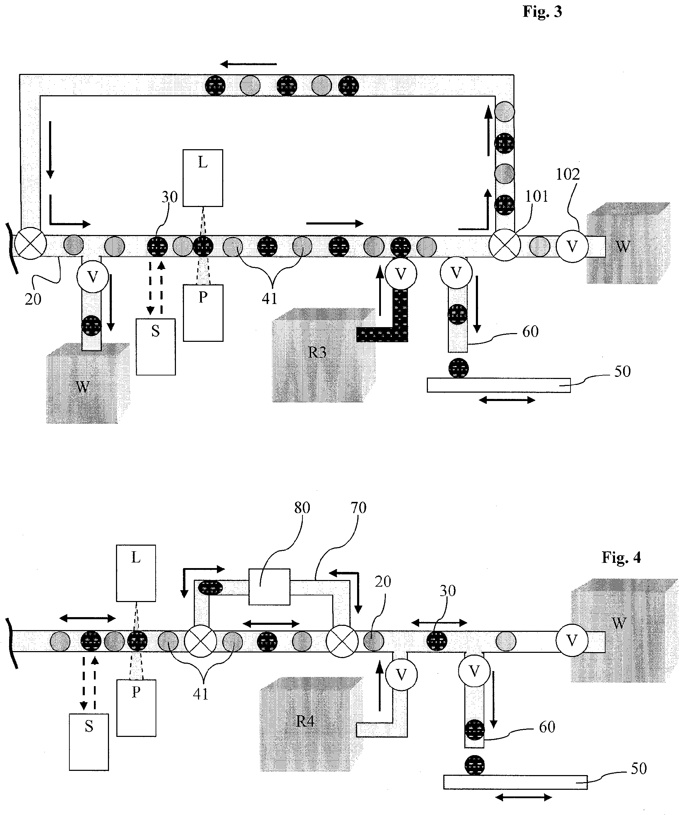

Le système réactionnel de cultures de microorganismes selon l'invention comprend avantageusement un ou plusieurs réservoirs R1, R2 de réactif 51, 52 reliés de manière fluidique au tube capillaire d'injection 10 par l'intermédiaire de connexions en T (voir figure 1) et/ou au tube capillaire de culture 20 (voir le réservoir R3 de la figure 3 relié directement au tube de culture 20), de sorte que du réactif 51, 52 puisse être mélangé au milieu de culture 1. Ceci permet de modifier la composition et/ou les propriétés chimiques et/ou physiques du milieu de culture, alors référencé 1'. Par exemple, il est possible d'enrichir ou d'appauvrir un réacteur 30 en éléments nutritifs, de modifier le pH, d'injecter des molécules de marquage, par exemple fluorescentes, d'injecter des molécules dont le pouvoir stimulant ou inhibant doit être testé sur les micro-organismes (par exemple des antibiotiques), etc.

Il est ainsi possible de définir la composition du milieu de culture de façon précise par un ajustement des débits des fluides composants la phase aqueuse.

Pour prévenir les risques de coalescence et des difficultés de détection liés au rapprochement des réservoirs 30, l'invention propose avantageusement d'intercaler un autre fluide non miscible avec le milieu de culture 1, 1' et avec le fluide porteur 21.

Ainsi, après l'étape c) et avant l'étape e), l'invention prévoit une étape d) comprenant l'injection, dans le fluide porteur 21, d'une goutte d'un fluide dit « de séparation », non miscible avec le fluide porteur et non miscible avec le milieu de culture, de sorte qu'au moins une goutte 40 de fluide de séparation soit intercalée entre deux réacteurs de culture 30 et empêche leur coalescence.

A cette fin, le système réactionnel de cultures de micro-organismes selon l'invention, comprend avantageusement au moins un réservoir F2 d'un fluide 41 de séparation non miscible avec le fluide porteur 21 et non miscible avec le milieu de culture 1, 1'.

Ce réservoir F2 est relié de manière fluidique au tube capillaire de culture 20 par l'intermédiaire d'une vanne à deux voies 102 de sorte que des gouttes 40 de fluide de séparation puissent être injectées dans le fluide porteur 21 entre deux réacteurs de culture 30.

Le fluide porteur 21 et le fluide de séparation 41 sont, de préférence, des huiles non miscibles entre elles, comme par exemple l'huile fluorée comme fluide porteur et l'huile minérale pour le fluide de séparation, le milieu de culture 1, 1' étant un milieu aqueux non miscible avec les huiles 21, 41 précitées.

La longueur du tube capillaire de culture, dans lequel sont formées les réacteurs, et les débits imposés définissent la quantité de réacteurs qui peuvent être utilisés par expérience et le pas de temps entre chaque mesure. Il est ainsi possible de travailler sur plusieurs milliers de réacteurs en parallèle. Cette méthode de manipulation de gouttes à une dimension permet de conserver l'identité de chaque goutte au cours d'une expérience, de maitriser parfaitement leur composition en évitant toute perte par évaporation ou reversement.

Selon un mode de réalisation préféré, le procédé selon l'invention comprend, après l'étape e), une étape f) de mesure d'un ou plusieurs paramètres représentatifs de chaque réacteur de culture 30, ce paramètre pouvant être représentatif, par exemple, de la quantité de micro-organismes présents dans chaque réacteur.

La mesure peut être réalisée par un procédé optique tel que les mesures d'absorbance, de diffusion ou de fluorescence, ou par une mesure électrique telle que l'impédance.

Un premier mode de réalisation d'une deuxième partie fonctionnelle du système réactionnel selon l'invention est illustrée en figure 3. Cette deuxième partie fonctionnelle permet de mettre en oeuvre les étapes précitées du procédé.

A cette fin, le tube capillaire de culture 20, au moins, comprend au moins une partie transparente à un signal émis et/ou capté par un détecteur S, ou un détecteur L-P.

Le détecteur S peut être un capteur électrique d'impédance.

Le détecteur L-P est constitué, dans cet exemple, d'un laser L émettant un rayonnement optique d'excitation, et d'une photodiode P sensible au rayonnement émis par le réservoir 30 sous l'excitation du laser pouvant être placée dans l'axe ou à un angle du rayonnement d'excitation (dans le cas de la diffusion de la lumière on peut observer la lumière diffusée par la goutte à 90° du laser ou tout autre angle).

Le mode de réalisation illustré en figure 3 comprend un moyen de recirculation des réservoirs comprenant une boucle de recirculation des réacteurs devant le ou les détecteurs S, L-P. Cette boucle de recirculation comprend alors un capillaire débouchant en amont et en aval du ou des détecteurs S, L-P. Dans ce cas le moyen de mise en circulation des fluides (milieu de culture, fluide porteur) peut fonctionner uniquement dans un seul sens de circulation.

Il est ainsi possible de suivre la croissance des micro-organismes dans chaque réservoir en déplaçant mécaniquement le fluide porteur, faisant ainsi passer et repasser (recirculation des réacteurs) chaque réacteur devant le ou les détecteur(s) S ou L-P, dans le même sens de circulation. Ce déplacement est très facile à mettre en oeuvre, et ne risque pas de renverser les réacteurs, comme avec les plaques de l'état de l'art. Ainsi, la vitesse de déplacement peut être accélérée, d'autant plus s'il y a des gouttes de fluide de séparation 40 entre chaque réacteur 30.

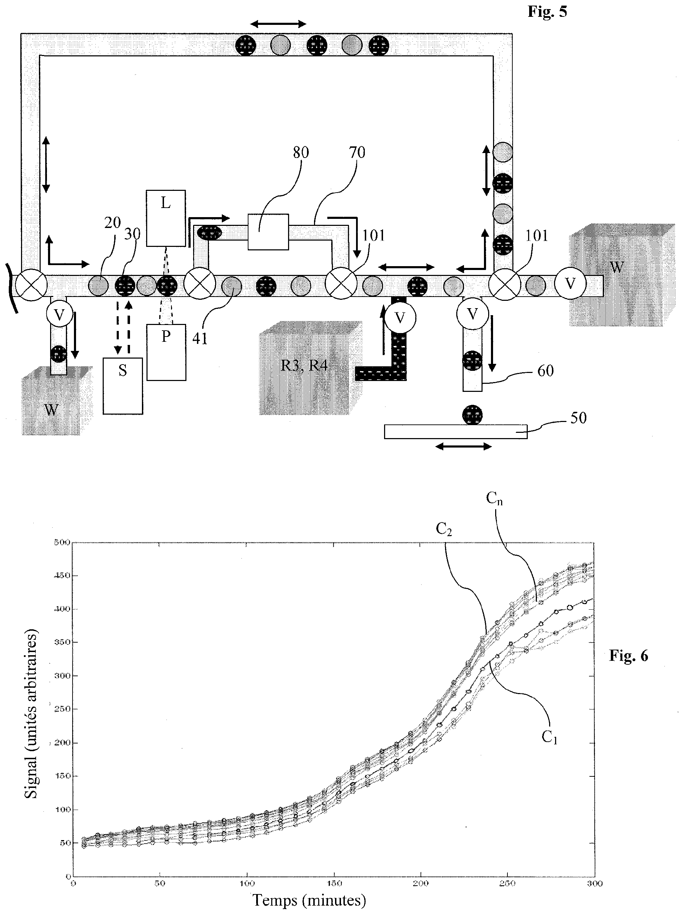

Alternativement, comme illustré en figure 4, ou en combinaison, comme illustré en figure 5, le moyen de mise en circulation des fluides peut fonctionner de manière bidirectionnelle, c'est-à-dire en entrainant les fluides dans les capillaires dans un sens ou dans le sens opposé.

Il est ainsi possible de suivre la croissance des micro-organismes dans chaque réservoir en déplaçant mécaniquement le fluide porteur selon des allers-retours, pour faire recirculer chaque réacteur devant le ou les détecteur(s) S ou L-P.

La combinaison d'une boucle de recirculation et d'un moyen de mise en circulation des fluides fonctionnant de manière bidirectionnelle (figure 5) permet de réduire le temps entre deux passages d'un même réacteur devant le ou les détecteurs, en optimisant la recirculation en fonction des distances amont et aval du réservoir par rapport aux détecteurs. Ainsi, selon le besoin de l'utilisateur et selon le cas, il sera avantageux d'inverser le sens de circulation du fluide pour ramener un réacteur d'intérêt devant le ou les détecteurs S ou L-P, ou, au contraire, de poursuivre la circulation du fluide porteur dans le même sens, pour faire recirculer le réacteur d'intérêt via la boucle de recirculation.

En connaissant précisément la place de chaque réacteur dans le train de réacteurs, et en connaissant les caractéristiques chimiques et/ou physiques du milieu de culture, il est possible d'analyser l'influence de la composition du milieu de culture sur la croissance des micro-organismes, Des courbes de mesure C₁, C₂, Cₙ de la population de micro-organismes dans une pluralité de réacteurs est illustrée en figure 6.

Il est ainsi possible de mesurer la variation de la population des micro-organismes dans chaque réservoir successif au cours du temps en pratiquant des passages successifs des réservoirs, dans le même sens de circulation (figures 3 et 5) et/ou en alternant les sens de circulation (figures 4 et 5), devant les détecteurs. Chaque courbe représente un réacteur identifié et chaque point représente le passage du réacteur considéré devant le détecteur à un intervalle de temps donné. Ainsi chaque goutte est identifiée, mesurée et paramétrée.

Un réservoir R4 de fluide porteur peut être monté débouchant dans le capillaire de culture 20 pour séparer les gouttes avant des les trier (figure 4). Ce réservoir R4 peut, bien entendu, être prévu en combinaison avec un ou plusieurs réservoirs R3 de réactifs tels que celui illustré aux figures 3 et 5.

Comme illustré aux figures 3 à 5, le trieur de culture de micro-organismes selon l'invention comprend de préférence au moins un réservoir de déchet W, en liaison fluidique avec le tube capillaire de culture 20 pour évacuer les réacteurs après l'expérimentation et des vannes 101-102 pour orienter les gouttes.

L'invention permet également facilement de sélectionner et d'extraire un réacteur de culture d'intérêt. Ainsi, l'invention propose, après l'étape e), une étape f') de récupération d'au moins un réacteur de culture d'intérêt par aspiration dudit réacteur d'intérêt dans un tube capillaire de prélèvement 60 monté débouchant dans le tube capillaire de culture 20.

Avantageusement, le tube capillaire de prélèvement permet le dépôt du réacteur prélevé sur un support de culture 65, tel qu'une couche d'agar ou dans les puits d'une microplaque.

Ainsi, lors de la détection, chaque réacteur est labellisé par sa position dans le train, et un détecteur de comptage permet d'identifier les réservoirs. Au moyen de vannes 102, le ou les réacteurs d'intérêt sont orientés dans le tube capillaire de prélèvement 60, tandis que les autres réacteurs restent dans le tube capillaire de culture 20. Le ou les réacteurs d'intérêt sont récupérés en sortie du tube capillaire de prélèvement 60.

L'inverse est également possible : les réacteurs d'intérêt sont conservés dans le tube capillaire de culture 20, alors que les autres réacteurs sont prélevés et éliminés. Il ne reste alors dans le tube de culture 20 que les réacteurs d'intérêt pour l'expérimentation.

Alternativement, ou en combinaison, l'invention prévoit avantageusement au moins un tube capillaire de dérivation 70 monté débouchant dans le tube capillaire de culture 20 de sorte qu'au moins un réacteur 30 de culture puissent être dérivé vers un moyen de traitement 80 d'un ou plusieurs réacteurs 30. Ce moyen de traitement 80 peut être un moyen de régulation thermique pouvant chauffer ou refroidir un ou plusieurs réacteurs. D'autres moyens de traitement peuvent être prévus tels que l'ajout de milieu de culture ou le prélèvement d'une portion du réacteur permettant la réalisation d'un chémostat (bioréacteur dans lequel poussent, de façon controlée, des organismes (bactéries, phytoplancton)).

Le tube capillaire de dérivation 70 permet ainsi un traitement sélectif d'un ou plusieurs réacteurs par rapport aux autres réacteurs restés dans le tube de culture 20. On comprend que plusieurs tubes de dérivations peuvent être prévus pour traiter de manière différente des réacteurs 30 dérivés sélectivement.

L'invention est avantageusement mise en oeuvre par l'intermédiaire d'une unité centrale de contrôle reliée au moyen de mise en circulation et apte à :
- commander l'injection de gouttes individuelles de milieu de culture dans le tube capillaire de culture, au sein du fluide porteur, en imposant une vitesse et une durée de circulation du milieu de culture, de manière à former un pluralité de réacteurs 30 de culture de micro-organismes ;
- commander la circulation du fluide porteur en imposant une vitesse, une durée et un sens de circulation du fluide porteur dans le tube capillaire de culture par le contrôle des vannes;
- compter les réacteurs 30 de culture dans le milieu porteur et mémoriser la place de chaque réacteur par rapport à un réacteur 30 de référence ;
- faire recirculer les réservoirs (30) dans le tube capillaire de réaction.
- commander l'injection de gouttes 41 de fluide de séparation entre deux réacteurs 30 de culture ;
- commander l'injection d'au moins un réactif 51, 52 au sein du milieu de culture 1, 1' pour en modifier la composition et/ou les propriétés chimiques et/ou physiques ;
- commander le prélèvement d'au moins un réacteur d'intérêt ; et/ou
- commander la dérivation d'au moins un réacteur d'intérêt vers un moyen de traitement, lui-même avantageusement commandé par l'unité centrale.

Avantageusement, l'unité centrale de contrôle est, en outre, reliée au détecteur S, L-P et est apte à mémoriser au moins une mesure réalisée par le détecteur.

Avantageusement, le système réactionnel comprend un moyen de régulation thermique des réacteurs qui est, de préférence, agencé pour permettre une régulation thermique dans tout le système réactionnel. Cette régulation thermique peut être homogène, c'est-à-dire sensiblement identique dans tout le système, où hétérogène, c'est-à-dire que la température peut être augmentée à certains endroits et diminuée à d'autre endroits du système.

## Revendications

1. Système réactionnel, notamment de cultures de micro-organismes, comprenant :
• au moins un réservoir (M1) de milieu réactionnel (1, 1') relié de manière fluidique à un tube capillaire d'injection (10) ;
• au moins un réservoir (F1) d'un fluide porteur (11) non miscible avec le milieu réactionnel (1, 1', 51, 52), relié de manière fluidique à un tube capillaire de réaction (20),
• le tube capillaire d'injection (10) étant monté débouchant dans le tube capillaire de réaction (20) de sorte que des gouttes individuelles (30) de milieu réactionnel puissent être injectées dans le tube capillaire de réaction (20), au sein du fluide porteur (11) non miscible, de manière à former un train de réacteurs,
• au moins un détecteur de suivi de réaction,
**caractérisé en ce qu'**il comprend un moyen de référencement des réacteurs pour les identifier de manière unique dans le train de réacteurs, et au moins un moyen de mise en recirculation des réacteurs devant au moins un détecteur de suivi de réaction.

2. Système réactionnel selon la revendication 1, dans lequel le moyen de mise en recirculation comprend une boucle de recirculation des réacteurs devant le ou les détecteurs (S, L-P), cette boucle de recirculation comprenant un capillaire débouchant en amont et en aval du ou des détecteurs (S, L-P).

3. Système réactionnel selon la revendication 1 ou 2, dans lequel le moyen de mise en recirculation comprend un moyen de mise en recirculation apte à inverser le sens de circulation des réacteurs.

4. Système réactionnel selon l'une quelconque des revendications 1 à 3, pour la culture de micro-organismes, dans lequel le milieu réactionnel est un milieu de culture de microorganismes, le tube capillaire de réaction est un tube capillaire de culture, et les réacteurs sont des réacteurs de culture de micro-organismes.

5. Système réactionnel selon l'une quelconque des revendications 1 à 4, comprenant, en outre, au moins un réservoir (F2) d'un fluide (41) dit « de séparation » non miscible avec le fluide porteur (21) et non miscible avec le milieu réactionnel (1, 1'), relié de manière fluidique au tube capillaire de réaction (20) de sorte que des gouttes (40) de fluide de séparation puissent être injectées dans le fluide porteur (21) entre deux réacteurs (30).

6. Système réactionnel selon l'une quelconque des revendications 1 à 5, comprenant, en outre, au moins un réservoir (R1, R2) de réactif (51, 52) relié de manière fluidique au tube capillaire d'injection (10) et/ou au tube capillaire de réaction (20), de sorte que du réactif puisse être mélangé au milieu réactionnel.

7. Système réactionnel selon l'une quelconque des revendications 5 ou 6, dans lequel le fluide porteur (21) et le fluide de séparation (41) sont des huiles non miscibles entre elles, le milieu réactionnel (1, 1') étant un milieu aqueux non miscible avec les huiles (21, 41) précitées.

8. Système réactionnel selon l'une quelconque des revendications 1 à 7, comprenant, en outre, au moins un réservoir de déchet (W), en liaison fluidique avec le tube capillaire de réaction (20).

9. Système réactionnel selon l'une quelconque des revendications 1 à 8, comprenant, en outre, au moins un détecteur (S, L-P), le tube capillaire de réaction comprenant au moins une partie transparente à un signal émis et/ou capté par le détecteur.

10. Système réactionnel selon l'une quelconque des revendications 1 à 9, comprenant, en outre, un tube capillaire de prélèvement (60) monté débouchant dans le tube capillaire de réaction (20) de sorte qu'au moins un réacteur (30) puisse être prélevé.

11. Système réactionnel selon l'une quelconque des revendications 1 à 10, comprenant, en outre, au moins un tube capillaire de dérivation (70) monté débouchant dans le tube capillaire de réaction (20) de sorte qu'au moins un réacteur (30) puissent être dérivé vers un moyen de traitement d'un ou plusieurs réacteurs (30).

12. Système réactionnel selon l'une quelconque des revendications 1 à 11, comprenant, en outre, une unité centrale de contrôle reliée au moyen de mise en circulation et apte à :
• commander l'injection de gouttes individuelles de milieu réactionnel dans le tube capillaire de réaction, au sein du fluide porteur, en imposant une vitesse et une durée de circulation du milieu de réactionnel, de manière à former une pluralité de réacteurs (30) ;
• commander la circulation du fluide porteur en imposant une vitesse, une durée et un sens de circulation du fluide porteur dans le tube capillaire de réaction ;
• compter les réacteurs (30) dans le milieu porteur et mémoriser la place de chaque réacteur par rapport à un réacteur (30) de référence ;
• faire recirculer les réservoirs (30) dans le tube capillaire de réaction.

13. Système réactionnel selon les revendications 5 et 12, dans lequel l'unité centrale de contrôle est apte à commander l'injection de gouttes (41) de fluide de séparation entre deux réacteurs (30).

14. Système réactionnel selon les revendications 6 et 12, ou 6 et 13, dans lequel l'unité centrale de contrôle est apte à commander l'injection d'au moins un réactif (51, 52) au sein du milieu réactionnel (1, 1') pour en modifier la composition et/ou les propriétés chimiques et/ou physiques.

15. Système réactionnel selon les revendications 1 et 11, dans lequel l'unité centrale de contrôle est, en outre, reliée au détecteur (S, L-P) et est apte à mémoriser au moins une mesure réalisée par le ou les détecteur(s).

16. Procédé de suivi d'une réaction dans un milieu réactionnel, **caractérisé en ce qu'**il comprend les étapes suivantes :
a) remplir un tube capillaire (20) de réaction avec un milieu porteur (21) non miscible avec le milieu réactionnel (1,1');
b) injecter, grâce à un tube capillaire d'injection (10), une goutte individuelle (30) de milieu réactionnel dans le tube capillaire de réaction (20), au sein du fluide porteur (21) non miscible ;
c) mettre en circulation le fluide porteur (21) pour que la goutte de milieu réactionnel se déplace par rapport au tube capillaire d'injection ;
e) répéter les étapes b) et c) pour créer un train ordonné de gouttes de milieu réactionnel au sein du fluide porteur pour former une pluralité de réacteurs (30) ;
f) mesurer au moins un paramètre représentatif de chaque réacteur ;
g) faire recirculer au moins un réacteur pour mesurer ledit au moins un paramètre représentatif au cours du temps.

17. Procédé de suivi d'une réaction dans un milieu réactionnel selon la revendication 16, pour la culture de micro-organismes dans un milieu de culture, comprenant les étapes suivantes :
a) remplir un tube capillaire (20) de culture avec un milieu porteur (21) non miscible avec le milieu de culture (1,1');
b) injecter, grâce à un tube capillaire d'injection (10), une goutte individuelle (30) de milieu de culture dans le tube capillaire de culture (20), au sein du fluide porteur (21) non miscible ;
c) mettre en circulation le fluide porteur (21) pour que la goutte de milieu de culture se déplace par rapport au tube capillaire d'injection ;
e) répéter les étapes b) et c) pour créer un train ordonné de gouttes de milieu de culture au sein du fluide porteur pour former une pluralité de réacteurs (30) de culture de micro-organismes ;
f) mesurer au moins un paramètre représentatif de chaque réacteur de culture, ce paramètre pouvant être représentatif de la quantité de micro-organismes présents dans chaque réacteur ;
g) faire recirculer au moins un réacteur pour mesurer ledit au moins un paramètre représentatif au cours du temps.

18. Procédé de suivi d'une réaction dans un milieu réactionnel selon l'une quelconque des revendications 16 ou 17, comprenant, en outre, après l'étape c) et avant l'étape e), une étape d) comprenant l'injection, dans le fluide porteur (21), d'une goutte d'un fluide dit «de séparation », non miscible avec le fluide porteur et non miscible avec le milieu réactionnel, de sorte qu'au moins une goutte (40) de fluide de séparation soit intercalée entre deux réacteurs (30) et empêche leur coalescence.

19. Procédé de suivi d'une réaction dans un milieu réactionnel selon l'une quelconque des revendications 16 à 18, dans lequel la mesure est réalisée par un procédé optique tel que des mesures d'absorbance, de diffusion ou de fluorescence, ou par une mesure électrique telle que l'impédance.

20. Procédé de suivi d'une réaction dans un milieu réactionnel selon l'une quelconque des revendications 16 à 19, comprenant, en outre, après l'étape e), une étape f') de récupération d'au moins un réacteur d'intérêt par aspiration dudit réacteur d'intérêt dans un tube capillaire de prélèvement monté débouchant dans le tube capillaire de réaction.

## Patentansprüche

1. Reaktionssystem, insbesondere für Kulturen von Mikroorganismen, aufweisend:
• mindestens einen Behälter (M1) für ein Reaktionsmedium (1, 1'), der mit einem Spritz-Kapillarrohr (10) fluidverbunden ist,
• mindestens einen Behälter (1) für ein Trägerfluid (11), welches nicht mit dem Reaktionsmedium (1, 1', 51, 52) mischbar ist, der mit einem Reaktions-Kapillarrohr (20) fluidverbunden ist,
• wobei das Spritz-Kapillarrohr (10) in das Reaktions-Kapillarrohr (20) mündend montiert ist, so dass einzelne Tropfen (30) des Reaktionsmediums innerhalb des nichtmischbaren Trägerfluids (11) in das Reaktions-Kapillarrohr (20) gespritzt werden können, um eine Folge von Reaktoren zu bilden,
• mindestens einen Reaktionsüberwachungsdetektor,
**dadurch gekennzeichnet, dass** es aufweist: ein Mittel zum Referenzieren der Reaktoren, um diese auf einzigartige Weise in der Folge von Reaktoren zu identifizieren, und mindestens ein Mittel zum Rückführen der Reaktoren vor mindestens einem Reaktionsüberwachungsdetektor.

2. Reaktionssystem gemäß Anspruch 1, wobei das Mittel zum Rückführen eine Schleife zum Rückführen der Reaktoren vor dem oder den Detektoren (S, L-P) aufweist, wobei die Rückführschleife ein Kapillarrohr aufweist, das stromaufwärts und stromabwärts von dem oder den Detektoren (S, L-P) ausmündet.

3. Reaktionssystem gemäß Anspruch 1 oder 2, wobei das Mittel zum Rückführen ein Mittel zum Rückführen aufweist, das in der Lage ist, die Zirkulationsrichtung der Reaktoren umzukehren.

4. Reaktionssystem gemäß irgendeinem der Ansprüche 1 bis 3 für die Kultur von Mikroorganismen, wobei das Reaktionsmedium ein Kulturmedium für Mikroorganismen ist, das Reaktions-Kapillarrohr ein Kultur-Kapillarrohr ist und die Reaktoren Kultur-Reaktoren für Mikroorganismen sind.

5. Reaktionssystem gemäß irgendeinem der Ansprüche 1 bis 4, ferner aufweisend mindestens einen Behälter (F2) für ein als "Trennfluid" bezeichnetes Fluid (41), das nicht mit dem Trägerfluid (21) mischbar ist und nicht mit dem Reaktionsmedium (1, 1') mischbar ist, der mit dem Reaktions-Kapillarrohr (20) fluidverbunden ist, so dass Tropfen (40) von Trennfluid zwischen zwei Reaktoren (30) in das Trägerfluid (21) gespritzt werden können.

6. Reaktionssystem gemäß irgendeinem der Ansprüche 1 bis 5, ferner mindestens einen Behälter (R1, R2) für ein Reagenz (51, 52) aufweisend, der mit dem Spritz-Kapillarrohr (10) und/oder dem Reaktions-Kapillarrohr (20) fluidverbunden ist, so dass Reagenz im Reaktionsmedium gemischt werden kann.

7. Reaktionssystem gemäß irgendeinem der Ansprüche 5 oder 6, wobei das Trägerfluid (21) und das Trennfluid (41) Öle sind, die nicht miteinander mischbar sind, wobei das Reaktionsmedium (1, 1') ein wässriges Medium ist, das nicht mit den vorgenannten Ölen (21, 41) mischbar ist.

8. Reaktionssystem gemäß irgendeinem der Ansprüche 1 bis 7, ferner mindestens einen Behälter für Abfallstoffe (W) aufweisend, der mit dem Reaktions-Kapillarrohr (20) fluidverbunden ist.

9. Reaktionssystem gemäß irgendeinem der Ansprüche 1 bis 8, ferner mindestens einen Detektor (S, L-P) aufweisend, wobei das Reaktions-Kapillarrohr mindestens einen für ein von dem Detektor emittiertes und/oder eingefangenes Signal durchlässigen Teil aufweist.

10. Reaktionssystem gemäß irgendeinem der Ansprüche 1 bis 9, ferner ein Entnahme-Kapillarrohr (60) aufweisend, das in das Reaktions-Kapillarrohr (20) mündet, so dass mindestens ein Reaktor (30) entnommen werden kann.

11. Reaktionssystem gemäß irgendeinem der Ansprüche 1 bis 10, ferner mindestens ein Ableitungs-Kapillarrohr (70) aufweisend, das in das Reaktions-Kapillarrohr (20) mündend montiert ist, so dass mindestens ein Reaktor (30) in Richtung zu einem Bearbeitungsmittel für einen oder mehrere Reaktoren (30) abgeleitet werden kann.

12. Reaktionssystem gemäß irgendeinem der Ansprüche 1 bis 11, ferner eine zentrale Steuereinheit aufweisend, die mit dem Zirkulationsmittel verbunden ist und in der Lage ist,
• das Spritzen von einzelnen Tropfen von Reaktionsmedium in das Reaktions-Kapillarrohr innerhalb des Trägerfluids zu steuern, wobei eine Zirkulationsgeschwindigkeit und -dauer für das Reaktionsmedium festgelegt werden, um eine Mehrzahl von Reaktoren (30) zu bilden,
• das Zirkulieren des Trägerfluids zu steuern, wobei eine Geschwindigkeit, eine Dauer und eine Richtung der Zirkulation des Trägerfluids im Reaktions-Kapillarrohr festgelegt werden,
• die Reaktoren (30) im Trägermedium zu zählen und die Position jedes Reaktors bezüglich eines Referenzreaktors (30) zu speichern,
• die Behälter (30) in dem Reaktions-Kapillarrohr zurückzuführen.

13. Reaktionssystem gemäß den Ansprüchen 5 und 12, wobei die zentrale Steuereinheit in der Lage ist, das Spritzen von Tropfen (41) von Trennfluid zwischen zwei Reaktoren (30) zu steuern.

14. Reaktionssystem gemäß den Ansprüchen 6 und 12 oder 6 und 13, wobei die zentrale Steuereinheit in der Lage ist, das Spritzen mindestens eines Reagenzes (51, 52) innerhalb des Reaktionsmediums (1, 1') zu steuern, um dessen Zusammensetzung und/oder chemische und/oder physikalische Eigenschaften zu verändern.

15. Reaktionssystem gemäß den Ansprüchen 1 und 11, wobei die zentrale Steuereinheit ferner mit dem Detektor (S, L-P) verbunden ist und in der Lage ist, mindestens eine Messung zu speichern, die durch den oder die Detektoren realisiert wird.

16. Verfahren zum Überwachen einer Reaktion in einem Reaktionsmedium, **dadurch gekennzeichnet, dass** es die folgenden Schritte aufweist:
a) Füllen eines Reaktions-Kapillarrohres (20) mit einem nicht mit dem Reaktionsmedium (1, 1') mischbaren Trägermedium (21),
b) Spritzen, mit Hilfe eines Spritz-Kapillarrohres (10), eines einzelnen Tropfens (30) von Reaktionsmedium in das Reaktions-Kapillarrohr (20) innerhalb des nichtmischbaren Trägerfluids (21),
c) Zirkulieren des Trägerfluids (21), damit der Reaktionsmediumtropfen sich relativ zu dem Spritz-Kapillarrohr bewegt,
d) Wiederholen der Schritte b) und c), um eine geordnete Folge von Tropfen von Reaktionsmedium innerhalb des Trägerfluids zu erzeugen, um eine Mehrzahl von Reaktoren (30) zu bilden,
e) Messen mindestens eines Parameters, der für jeden Reaktor repräsentativ ist,
f) Rückführen mindestens eines Reaktors, um den mindestens einen repräsentativen Parameter über die Zeit zu messen.

17. Verfahren zum Überwachen einer Reaktion in einem Reaktionsmedium gemäß Anspruch 16 für die Kultur von Mikroorganismen in einem Kulturmedium, aufweisend die folgenden Schritte:
a) Füllen eines Kultur-Kapillarrohres (20) mit einem mit dem Kulturmedium (1, 1') nicht mischbaren Trägermedium (21),
b) Spritzen, mit Hilfe eines Spritz-Kapillarrohres (10), eines einzelnen Kulturmediumtropfens (30) in das Kultur-Kapillarrohr (20) innerhalb des nicht mischbaren Trägerfluids (21),
c) Zirkulieren des Trägerfluids (21), damit der Kulturmediumtropfen sich relativ zu dem Spritz-Kapillarrohr bewegt,
d) Wiederholen der Schritte b) und c), um eine geordnete Folge von Kulturmediumtropfen innerhalb des Trägerfluids zu erzeugen, um eine Mehrzahl von Reaktoren (30) für die Kultur von Mikroorganismen zu bilden,
e) Messen mindestens eines für jeden Kulturreaktor repräsentativen Parameters, wobei dieser Parameter für die Menge von in jedem Reaktor vorhandenen Mikroorganismen repräsentativ sein kann,
f) Rückführen mindestens eines Reaktors, um den mindestens einen repräsentativen Parameter über die Zeit zu messen.

18. Verfahren zum Überwachen einer Reaktion in einem Reaktionsmedium gemäß irgendeinem der Ansprüche 16 oder 17, ferner aufweisend, nach dem Schritt c) und vor dem Schritt e), einen Schritt d), der das Spritzen eines Tropfens eines als "Trennfluid" bezeichneten Fluids, das nicht mit dem Trägerfluid mischbar ist und nicht mit dem Reaktionsmedium mischbar ist, in das Trägerfluid (21) aufweist, so dass mindestens ein Tropfen (40) Trennfluid zwischen zwei Reaktoren (30) eingefügt wird und ihre Koaleszenz verhindert.

19. Verfahren zum Überwachen einer Reaktion in einem Reaktionsmedium gemäß irgendeinem der Ansprüche 16 bis 18, wobei das Messen durch ein optisches Verfahren, zum Beispiel Absorbanz-, Diffusions- oder Fluoreszenz-Messungen oder durch eine elektrische Messung, zum Beispiel die Impedanz realisiert wird.

20. Verfahren zum Überwachen einer Reaktion in einem Reaktionsmedium gemäß irgendeinem der Ansprüche 16 bis 19, ferner nach dem Schritt e) einen Schritt f') des Wiedergewinnens mindestens eines Reaktors von Interesse durch Ansaugen des Reaktors von Interesse in einem Entnahme-Kapillarrohr aufweisend, das in das Reaktions-Kapillarrohr mündend montiert ist.

## Claims

1. A reaction system, notably of cultures of microorganisms, comprising:
at least one reservoir (M1) of reaction mixture (1, 1') fluidically connected to a capillary injection tube (10) ;
at least one reservoir (F1) of a carrier fluid (11) that is immiscible with the reaction mixture (1, 1', 51, 52), fluidically connected to a capillary reaction tube (20),
the capillary injection tube (10) being mounted opening into the capillary reaction tube (20) so that individual drops (30) of reaction mixture are injectable into the capillary reaction tube (20), into the immiscible carrier fluid (11), so as to form a succession of reactors,
at least one reaction monitoring detector,
wherein the system comprises a means of referencing the reactors for identifying them uniquely in the succession of reactors, and at least one means for recirculating the reactors in front of at least one reaction monitoring detector.

2. The reaction system as claimed in claim 1, in which the recirculating means comprises a loop for recirculating the reactors in front of the detector or detectors (S, L-P), said recirculating loop comprising a capillary discharging upstream and downstream of the detector or detectors (S, L-P).

3. The reaction system as claimed in claim 1 or 2, in which the recirculating means comprises a recirculating means able to reverse the direction of circulation of the reactors.

4. The reaction system as claimed in one of the claims 1 to 3, for culturing microorganisms, in which the reaction mixture is a medium for culturing microorganisms, the capillary reaction tube is a capillary culture tube, and the reactors are reactors for culture of microorganisms.

5. The reaction system as claimed in one of the claims 1 to 4, further comprising at least one reservoir (F2) of a separating fluid (41) that is immiscible with the carrier fluid and immiscible with the reaction mixture (1, 1'), fluidically connected to the capillary reaction tube (20) so that drops (40) of separating fluid can be injected into the carrier fluid (21) between two reactors (30).

6. The reaction system as claimed in one of the claims 1 to 5, further comprising at least one reservoir (R1, R2) of reagent (R1, R2) fluidically connected to the capillary injection tube (10) and/or to the capillary reaction tube (20), so that reagent can be mixed with the reaction mixture.

7. The reaction system as claimed in claim 5 or 6, in which the carrier fluid (21) and the separating fluid (41) are mutually immiscible oils, the reaction mixture (1, 1') being an aqueous medium that is immiscible with the aforementioned oils (21, 41).

8. The reaction system as claimed in one of the claims 1 to 7, further comprising at least one waste reservoir (W) connected fluidically to the capillary reaction tube (20).

9. The reaction system as claimed in one of the claims 1 to 8, further comprising at least one detector (S, L-P), the capillary reaction tube comprising at least one portion that is transparent to a signal emitted and/or detected by the detector.

10. The reaction system as claimed in one of the claims 1 to 9, further comprising a sampling capillary tube (60) mounted opening into the capillary reaction tube (20) so that at least one reactor (30) can be taken.

11. The reaction system as claimed in one of the claims 1 to 10, further comprising at least one diverting capillary tube (70) mounted opening into the capillary reaction tube (20) so that at least one reactor (30) can be diverted to a means for treatment of one or more reactors (30).

12. The reaction system as claimed in one of the claims 1 to 11, further comprising a central control unit connected to the circulating means and configured for:
controlling the injection of individual drops of reaction mixture into the capillary reaction tube, into the carrier fluid, imposing a velocity and a duration of circulation of the reaction medium, so as to form a plurality of reactors (30);
controlling the circulation of the carrier fluid by imposing a velocity, duration and direction of circulation of the carrier fluid in the capillary reaction tube;
counting the reactors (30) in the carrier medium and storing the position of each reactor relative to a reference reactor (30);
recirculating the reservoirs (30) in the capillary reaction tube.

13. The reaction system as claimed in claims 5 and 12, in which the central control unit is capable of controlling the injection of drops of separating fluid between two reactors (30).

14. The reaction system as claimed in claims 6 and 12 or claims 6 and 13, in which the central control unit is capable of controlling the injection of at least one reagent (51, 52) into the reaction mixture (1, 1') for modifying at least one of its composition; its chemical and its physical properties.

15. The reaction system as claimed in claims 1 and 11, in which the central control unit is in addition connected to the detector (S, L-P) and is capable of storing at least one measurement performed by the detector or detectors.

16. A method of monitoring a reaction in a reaction mixture, wherein in the method comprises the following steps:
a) filling a capillary reaction tube (20) with a carrier medium (21) that is immiscible with the reaction mixture (1, 1');
b) injecting, by means of a capillary injection tube (10), an individual drop (30) of reaction mixture in the capillary reaction tube (20), into the immiscible carrier fluid (21);
c) circulating the carrier fluid (21) so that the drop of reaction mixture is moved relative to the capillary injection tube;
e) repeating steps b) and c) to create an ordered succession of drops of reaction mixture in the carrier fluid to form a plurality of reactors (30);
f) measuring at least one representative parameter of each reactor;
g) recirculating at least one reactor to measure said at least one representative parameter over time.

17. The method of monitoring a reaction in a reaction mixture as claimed in claim 16, for the culture of microorganisms in a culture medium, comprising the following steps:
a) filling a capillary culture tube (20) with a carrier medium (21) that is immiscible with the culture medium (1, 1');
b) injecting, by means of a capillary injection tube (10), an individual drop of culture medium (30) in the capillary culture tube (20), into the immiscible carrier fluid (21);
c) circulating the carrier fluid (21) so that the drop of culture medium is moved relative to the capillary injection tube;
e) repeating steps b) and c) to create an ordered succession of drops of culture medium in the carrier fluid to form a plurality of reactors (30) for culture of microorganisms;
f) measuring at least one representative parameter of each culture reactor, wherein said parameter can be representative of the quantity of microorganisms present in each reactor;
g) recirculating at least one reactor to measure said at least one representative parameter over time.

18. The method of monitoring a reaction in a reaction mixture as claimed in claim 16 or 17, further comprising, after step c) and before step e), a step d) comprising injection, into the carrier fluid (21), of a drop of a separating fluid, immiscible with the carrier fluid and immiscible with the reaction mixture, so that at least one drop (40) of separating fluid is interposed between two reactors (30) and prevents their coalescence.

19. The method of monitoring a reaction in a reaction mixture as claimed in one of the claims16 to 18, in which measurement is carried out by an optical method selected from the group consisting of measurements of absorbance, of diffusion, and of fluorescence, or by an electrical measurement comprising measuring impedance.

20. The method of monitoring a reaction in a reaction mixture as claimed in one of the claims 16 to 19, further comprising, after step e), a step f) of recovery of at least one reactor of interest by aspiration of said reactor of interest into a sampling capillary tube mounted opening into the capillary reaction tube.
